(19) **Europäisches Patentamt / European Patent Office / Office européen des brevets**

(11) **EP 3 385 738 A1**

(12) # EUROPEAN PATENT APPLICATION
published in accordance with Art. 153(4) EPC

(43) Date of publication:
**10.10.2018 Bulletin 2018/41**

(21) Application number: **16883967.8**

(22) Date of filing: **03.11.2016**

(51) Int Cl.:
*G01R 31/36* (2006.01)     *G01N 35/08* (2006.01)
*G08B 21/18* (2006.01)     *A61B 5/145* (2006.01)

(86) International application number:
**PCT/KR2016/012590**

(87) International publication number:
**WO 2017/119583 (13.07.2017 Gazette 2017/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **06.01.2016 KR 20160001478**

(71) Applicant: **Samsung Electronics Co., Ltd.**
**Suwon-si, Gyeonggi-do 16677 (KR)**

(72) Inventors:
• **LEE, Jung Tae**
  Suwon-si
  Gyeonggi-do 16542 (KR)
• **KIM, Jong Cheol**
  Seoul 04148 (KR)

(74) Representative: **D'Halleweyn, Nele Veerle Trees Gertrudis et al**
**Arnold & Siedsma**
**Bezuidenhoutseweg 57**
**2594 AC The Hague (NL)**

(54) **TESTING APPARATUS AND CONTROL METHOD THEREFOR**

(57)     Disclosed herein is a test apparatus and a control method thereof, particularly a test apparatus capable of inactivating an operation of an in vitro diagnostic device when it is impossible to perform a test, by allowing a user to intuitively recognize the current state of a battery provided in the in vitro diagnostic device by calculating a power consumption rate of a battery provided in the in vitro diagnostic device when an in vitro diagnostic device is used and determining a current amount of power of the battery based on the calculated power consumption rate, and a control method thereof. The test apparatus comprises a storage configured to store information related to a battery provided in the test apparatus, a controller configured to calculate a power consumption rate of the battery changed according to an operation of the test apparatus based on the stored information of battery and configured to determine a current amount of power of the battery based on the calculated power consumption rate of the battery; and a display configured to display the current amount of power of the battery and at least one piece of information related to the operation of the test apparatus based on the determined current amount of power of the battery.

[Fig. 5]

## Description

[Technical Field]

The present disclosure relates to a test apparatus configured to perform an in vitro diagnosis using a small amount of sample and a control method thereof.

[Background Art]

In Vitro Diagnostic is a technique to detect the health status of a patient by using blood and body fluid that can be separated from the patient, and is widely used as a pre-diagnosis for disease diagnosis. In general, an immunity test, a clinical chemistry test, and the like are performed on a patient's sample so as to perform an in vitro diagnosis. Thus, the immunity test and the clinical chemistry test play a very important role in a diagnosis, a treatment, and a prognosis determination of the patient's state.

A blood analyzer corresponding to a representative example of the in vitro diagnostic device is configured to test blood quickly and accurately by using test media, such as a disposable cartridge or a disc, and it is possible to check the health status of the patient using a small amount of blood that is collected from the patient blood.

A user uses test media to analyze a test object such as patient's blood and body fluids, by using an in vitro diagnostic device. As for the blood analyzer, a user puts sampling blood, which is collected from the patient, to the test media and then input the test media into the in vitro diagnostic device so that the blood analyzer analyzes the test object. The target medium may include a reagent for analyzing a target object. For example, the blood analyzer may identify whether the patient has a certain antigen or antibody, or whether the patient is infected with a certain disease, by analyzing the reaction between the reagent contained in the test medium and the target object. In addition, the blood analyzer is used for drug tests as well as the diagnosis of diseases.

Particularly, in the medical diagnosis, the reliance on a point-of-care (POC) blood analyzer that uses a disposable cartridge has been increased and thus a research and development of small POC blood analyzer capable of rapidly and accurately testing blood has been actively performed in the world.

A battery is used for the operation of the in vitro diagnostic device and thus it is necessary to accurately estimate a period of time in which the in vitro diagnostic device can operate and the number of times of the tests according to the amount of power of the battery. In addition, it is necessary to accurately estimate the battery life and battery characteristics change by calculating the battery manufacturing time, the number of times in which the battery is used, the power consumption rate according to the temperature of the battery, and the power consumption rate of the battery according to the use of the in vitro diagnostic device.

[Disclosure]

[Technical Problem]

The present disclosure is directed to providing a test apparatus allowing a user to intuitively recognize the current state of a battery provided in an in vitro diagnostic device and inactivate an operation of an in vitro diagnostic device when it is impossible to perform a test, by calculating a power consumption rate of a battery provided in the in vitro diagnostic device with use of the in vitro diagnostic device, determining the current amount of power of the batterybased on the calculated power consumption rate, and informing the user of the time in which the in vitro diagnostic device can operate and the number of times of possible tests, and a control method thereof.

[Technical Solution]

One aspect of the present disclosure provides a test apparatus configured to test a test medium accommodated in a reaction device,

the test apparatus including a storage configured to store information related to a battery provided in the test apparatus, a controller configured to calculate a power consumption rate of the battery changed according to an operation of the test apparatus based on the stored information of battery and configured to determine a current amount of power of the battery based on the calculated power consumption rate of the battery; and a display configured to display the current amount of power of the battery and at least one piece of information related to the operation of the test apparatus based on the determined current amount of power of the battery.

The information related to the operation of the test apparatus may include at least one of an available operation time and an available number of test times of the test apparatus based on the determined current amount of power of the battery.

The information of battery may include at least one of a power consumption rate based on a manufacturing date of the battery, the number of uses of the battery, a power consumption rate based on the number of uses of the battery, a power consumption rate based on a temperature of the battery, and a power consumption rate based on the type of test.

The controller may calculate the power consumption rate of the battery changed according to an operation of the test apparatus based on the test type of the test apparatus.

The controller may determine whether to proceed with the test of the test apparatus based on the determined current amount of power of the battery.

The controller may control the power needed for driving the test apparatus based on the determined current amount of power of the battery.

When the determined current amount of power of the battery is equal to or less than a predetermined amount of power, the controller may transmit a control signal configured to reduce the power consumption of the test apparatus.

When the determined current amount of power of the battery is equal to or less than a predetermined amount of power, the controller may cut off the power needed for a test progress of the test apparatus.

When the determined current amount of power of the battery is equal to or less than a predetermined amount of power, the controller may transmit a control signal configured to prevent a control screen for the test progress of the test apparatus from being displayed.

The display may display at least one of an available operation time and an available number of test times of the test apparatus based on the determined current amount of power of the battery.

The display may display at least one of an available operation time and an available number of test times of the test apparatus based on the test type of the test apparatus.

The display may display whether the test apparatus proceeds with the test, based on the determined current amount of power of the battery.

When the determined current amount of power of the battery is equal to or less than a predetermined amount of power, the display may not display a control screen for the test progress of the test apparatus.

The storage may store at least one of a power consumption rate based on a manufacturing date of the battery, the number of uses of the battery, a power consumption rate based on the number of uses of the battery, a power consumption rate based on a temperature of the battery, and a power consumption rate based on the type of test.

The storage may store at least one of the calculated power consumption rate of the battery and the current amount of power of the battery.

The test apparatus may further include a communicator configured to transmit a control signal to allow the current amount of power of the battery and at least one piece of information related to an operation of the test apparatus to be displayed on an external device, based on the determined current amount of power of the battery.

The test apparatus may further include a notifier configured to transmit a notification to a user when the determined current amount of power of the battery is equal to or less than a predetermined amount of power.

Another aspect of the present disclosure provides a control method of a test apparatus configured to test a test medium accommodated in a reaction device,

the control method including calculating a power consumption rate of the battery changed according to an operation of the test apparatus based on the stored information of battery, determining a current amount of power of the battery based on the calculated power consumption rate of the battery, and displaying the current amount of power of the battery and at least one piece of information related to the operation of the test apparatus based on the determined current amount of power of the battery.

The calculation of the power consumption rate of the battery may be performed by calculating the power consumption rate of the battery changed according to an operation of the test apparatus based on the test type of the test apparatus.

The control method may include determining whether to proceed with the test of the test apparatus based on the determined current amount of power of the battery.

The control method may include controlling the power needed for driving the test apparatus based on the determined current amount of power of the battery.

The control method may include transmitting a control signal configured to reduce the power consumption of the test apparatus when the determined current amount of power of the battery is equal to or less than a predetermined amount of power.

The control method may include cutting off the power needed for a test progress of the test apparatus when the determined current amount of power of the battery is equal to or less than a predetermined amount of power.

The control method may include transmitting a control signal configured to prevent a control screen for the test progress of the test apparatus from being displayed when the determined current amount of power of the battery is equal to or less than a predetermined amount of power.

The display of at least one piece of the information related to the operation of the test apparatus may include displaying at least one of an available operation time and an available number of test times of the test apparatus based on the determined current amount of power of the battery.

The display of at least one piece of the information related to the operation of the test apparatus may include displaying at least one of an available operation time and an available number of test times of the test apparatus based on the test type of the test apparatus.

The display of at least one piece of the information related to the operation of the test apparatus may include displaying whether the test apparatus proceeds with the test, based on the determined current amount of power of the battery.

The control method may include transmitting a control signal allowing the current amount of power of the battery and at least one of information related to an operation of the test apparatus to be displayed on an external device, based on the determined current amount of power of the battery.

The control method may include transmitting a notification to a user when the determined current amount of power of the battery is equal to or less than a predetermined amount of power.

[Advantageous Effects]

A user can intuitively recognize an available operation time and an available number of test times of an in vitro diagnostic device since the in vitro diagnostic device is capable of accurately calculating a power consumption rate of a battery according to an operation of the in vitro diagnostic device based on information related to the battery and informs the user of the available operation time and the available number of test times of the in vitro diagnostic device.

It is possible to prevent a test from being stopped during the test or to prevent test media from being wasted because the power is controlled or the test process is inactivated when the current amount of power of the battery is too low to proceed with the test of the in vitro diagnostic device.

[Description of Drawings]

FIG. 1 is an external view illustrating a test apparatus according to an embodiment.

FIG. 2 is an external view illustrating a reaction device inserted into the test apparatus of FIG. 1 according to an embodiment.

FIG. 3 is an external view illustrating a test apparatus according to another embodiment.

FIG. 4 is an external view illustrating a reaction device inserted into the test apparatus of FIG. 3 according to another embodiment.

FIG. 5 is a control block diagram illustrating a configuration of the test apparatus according to an embodiment.

FIG. 6 illustrates data on a power consumption rate based on the type of test apparatus and the number of uses of a battery according to an embodiment.

FIG. 7 illustrates data on a power consumption rate based on a manufacturing data of the battery and the temperature of the battery according to an embodiment.

FIG. 8 illustrates displaying of information related to a current power amount of the battery and the operation of the test apparatus according to an embodiment.

FIG. 9 illustrates displaying of information related to a current power amount of a battery and the operation of a test apparatus according another embodiment.

FIG. 10 is a screen illustrating a control of a power supply for driving the test apparatus according to an embodiment.

FIG. 11 is a screen illustrating that the power supplied for the test process of the test apparatus is cut off.

FIG. 12 is a screen illustrating that a control screen for the test process of the test apparatus is inactivated.

FIG. 13 is a view illustrating that the amount of power of the battery and at least one piece of information related to the operation of the test apparatus is displayed on an external device according to an embodiment.

FIG. 14 is a flowchart illustrating a method of controlling the test apparatus according to an embodiment.

FIG. 15 is a flowchart illustrating a method of controlling a test apparatus according to another embodiment.

[Mode for Invention]

Advantages and features of the present disclosure, and methods and apparatus for accomplishing them will become apparent with reference to the embodiments described below with reference to the accompanying drawings. The disclosure may, however, be embodied in many different forms and should not be construed as being limited to the embodiments set forth herein; rather, these embodiments are provided so that this disclosure will be thorough and complete, and will fully convey the concept of the disclosure to those skilled in the art

The terms used in the specification will be briefly described, and the present disclosure will be described in detail

All terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the disclosure. Thus, the terms used herein have to be defined based on the meaning of the terms together with the description throughout the specification.

When a part "includes" or "comprises" an element, unless there is a particular description contrary thereto, the part can further include other elements, not excluding the other elements. In the following description, terms such as "part", "module" and "unit" indicate a unit for processing at least one function or operation, wherein the unit and the block may be embodied as software or hardware, such as Field Programmable Gate Array (FPGA), Application Specific Integrated Circuit (ASIC), or embodied by combining hardware and software. However, the term "part" "module" and "unit" are not limited to software or hardware. Further, "part" "module" and "unit" may be constructed to exist in an addressable storage module, or to play one or more processors. "part" "module" and "unit" includes elements (e.g., software elements, object-oriented software elements, class elements and task elements), processors, functions, properties, procedures, subroutines, segments of a program code, drivers, firmware, a microcode, a circuit, data, a database, data structures, tables, arrays, and variables. Herein, functions provided by components and modules may be provided by a smaller number of combined larger components and modules, or by a larger number of divided smaller components and modules.

The present disclosure will now be described more fully with reference to the accompanying drawings, in which exemplary embodiments of the disclosure are shown. In the description of the present disclosure, if it is determined that a detailed description of commonly-used technologies or structures related to the embodiments of the present disclosure may unnecessarily obscure the subject matter of the disclosure, the detailed description will be omitted.

As used herein, the term "user" refers to a medical professional, such as a paramedic, a physician, a nurse, a clinical pathologist, a medical imaging specialist, etc., and a technician capable of repairing medical devices, but is not limited thereto.

Further, a test apparatus described below is not limited to an in vitro diagnostic device, but the test apparatus according to an embodiment of the disclosure will be described with the in vitro diagnostic device as an example.

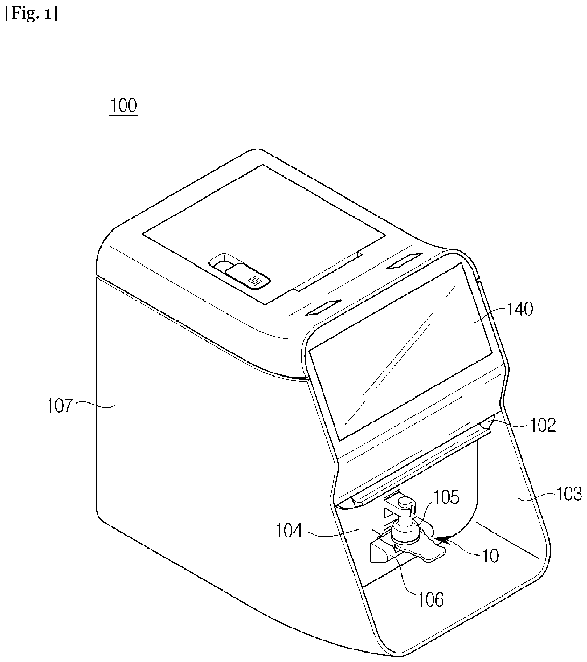

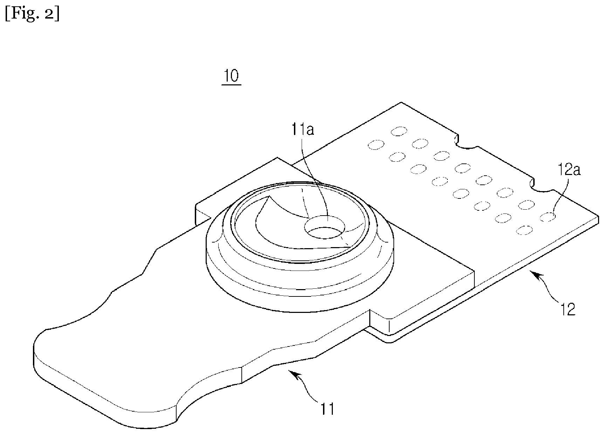

FIG. 1 is an external view illustrating a test apparatus according to an embodiment, and FIG. 2 is an external view illustrating a reaction device inserted into the test apparatus of FIG. 1 according to an embodiment.

A test apparatus 100 may be miniaturized and automated to test various samples, such as environmental samples, bio samples, and food samples. Particularly, when using a test apparatus for in vitro diagnosis of a bio sample collected from a human body, Point Of Care Testing (POCT) may be rapidly implemented in inspection rooms and other places, such as home, offices, clinics, hospital rooms, emergency rooms, operating rooms, and intensive care units, by users, including patients, doctors, nurses, and medical laboratory technicians.

On the other hand, a reaction device in which the sample is injected and the reaction between the reagent and the sample takes place includes a cartridge type in which a sample or a reagent moves by the capillary force, a disc type in which a sample or a reagent moves by the centrifugal force, and a cuvette type in which the measurement is immediately performed without moving a sample or a reagent. Depending on the type of reaction device, a structure and configuration of a test apparatus may vary, and an example of FIG. 1 shows a test apparatus to which a cartridge type reaction device is inserted.

Referring to FIG. 1, the test apparatus 100 may include a mounter 203 in which the reaction device 10 is mounted, and when a door 102 of the mounter 203 is slid upward to be opened, the reaction device 10 may be mounted on the test apparatus 100. For example, a part of the reaction device 10 may be inserted into a predetermined insertion groove 104 provided in the mounter 103.

The part of the reaction device 10 may be inserted into a main body 107, and a remaining part of the reaction device 10 may be exposed to an outside of the test apparatus 100 so as to be supported by a support 106. When a pressurizer 105 pressurizes the reaction device 10, the sample may be promoted to flow into a region in which the reaction takes place.

When the mounting of the reaction device 10 is completed, the test apparatus 100 closes the door 102 and stats a test.

The cartridge type reaction device inserted into the test apparatus 100 according to the example of FIG.1 may have an appearance as shown in FIG. 2.

Referring to FIG. 2, the reaction device 10 according to an embodiment may include a housing 11, and a platform 12 on which a reagent and a sample meet and the reaction takes place.

The housing 11 is configured to allow a user to grip the reaction device 10 while supporting the platform 12. The platform 12 may be coupled with the housing 11 by being joined to the bottom of the housing 11 or being inserted into a predetermined groove formed on the housing 11.

The housing 110 may be formed of a material that is easily formed and that is chemically and biologically inactive. For example, one of various materials, such as a plastic material, such as, acryl, such as polymethylmethacrylate (PMMA), polysiloxane, such as polydimethylsiloxane (PDMS), polycarbonate (PC), polyethylene, such as, linear low density polyethylene (LLDPE), low density polyethylene (LDPE), medium density polyethylene (MDPE), or high density polyethylene (HDPE), polyvinylalcohol (PVA), very low density polyethylene (VLDPE), polypropylene (PP), acrylonitrile butadien styrene (ABS), cyclo olefin copolymer (COC), glass, mica, silica, or a semiconductor wafer, may be used to form the housing 11.

An inlet hole 11a through which the sample flows is formed in the housing 11. A user may drop a sample corresponding to a test object to the inlet hole 11a by using a pipet or a dropping pipet.

A plurality of chambers 12a are formed in the platform 12, and a reagent is accommodated in the chambers 12a. For example, the reagent may be accommodated by being applied and dried on the inside of the chamber 12a. The sample flows into the inflow hole 11a reaches the chamber 12a through a channel (not shown) connecting the inlet hole 11a to the chamber 12a, and the sample reacts with a reagent previously accommodated in the chamber 12a. As illustrated in FIG. 1, a part of the reaction device 10 is inserted into the insertion groove 104 of the test apparatus 100. Since the reagent and the sample react in the chamber 12a, the platform 12 may be inserted into the groove 104, and the pressurizer 105 may facilitate the inflow of the sample by pressurizing the inlet hole 11a.

Although not shown in the drawings, the flat form 12 may have a structure in which three plates are bonded to one another. Three plates may be divided into an upper plate, a lower plate and a middle plate. The upper plate and the lower plate that are printed with a shielding ink may protect the sample that is moved to the chamber 12a, from external light.

The upper plate and the lower plate may be formed of films, and the films used to form the upper plate and the lower plate may be one selected from a polyethylene film, such as VLDPE, LLDPE, LDPE, MDPE, or HDPE, a PP film, a polyvinyl chloride (PVC) film, a PVA film, a polystyrene (PS) film, and a polyethylene terephthalate (PET) film.

The middle plate may be formed of a porous sheet, such as cellulose, and the middle plate itself may serve as a vent, and the porous sheet may be formed of a material having hydrophobicity, or hydrophobic treatment may be performed on the porous sheet so that the porous sheet may not affect movement of the sample.

When the flat form 12 has a triple layer structure, a hole forming the inlet hole 11a may be disposed between the upper plate and the middle plate, and a portion corresponding to the chamber 12a of the upper plate and the lower plate may be processed transparently. The portion corresponding to the chamber 12a of the upper plate and the lower plate is processed transparently to measure optical property caused by the reaction that takes place in the chamber 12a.

A thin channel is formed on the middle plate, and a sample introduced through the inlet hole 11a may move to the chamber 12a by the capillary force of the channel.

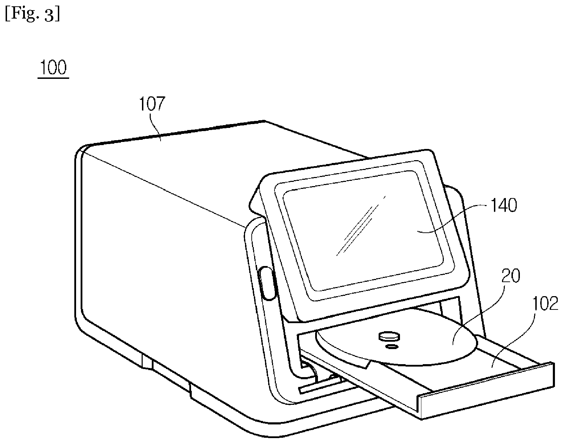

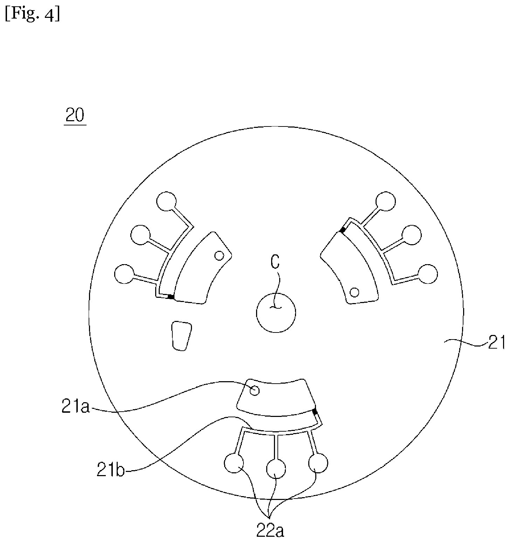

FIG. 3 is an external view illustrating a test apparatus according to another embodiment, and FIG. 4 is an external view illustrating a reaction device inserted into the test apparatus of FIG. 3 according to another embodiment.

The example of FIG. 3 relates to a test apparatus into which a disk type reaction device is inserted.

Referring to FIG. 3, a test apparatus 100 may include a tray 102 on which a disk type reaction device 20 is placed be placed. The placed reaction device 20 may be inserted into a main body 107 of the test apparatus 100 together with the tray 102. When the reaction device 20 is inserted into the main body 107, the test apparatus 100 rotates the reaction device 20 according to a predetermined sequence according to the type of the reaction device 20 inserted into the test apparatus 100 or the kind of the sample or the test process, and measures a test result.

Referring to FIG. 4, the disk type reaction device 20 may include a rotatable platform 21, and structures disposed on the platform 21. The structures include a plurality of chambers in which a sample or reagent is accommodated, and a channel that connects the plurality of chambers. The structures are formed in the reaction device 20. However, in the current embodiment, the reaction device 20 is formed of a transparent material, and when the reaction device 20 is viewed from the above, the structures formed in the reaction device 20 may be seen.

The platform 21 may be formed of a material which is easily formed and of which surface is biologically inactive. For example, the platform 21 may be formed of one of various materials, such as a plastic material, such as, PMMA, PDMS, PC, PP, PVA, or PE, glass, mica, silica, and a silicon wafer.

However, embodiments of the present disclosure are not limited thereto. Any type of material having chemical and biological stability and mechanical processability may be the material of the platform 21, and when a test result within the reaction device 20 is optically analyzed, the platform 21 may further have optical transparency.

The platform 21 may be provided with an inlet hole 21a through which a sample flows, a chamber 22a through which a reagent is previously accommodated , and a channel 21b connecting the inlet hole 21a to the chamber 22a.

As mentioned in FIG. 3, the test apparatus 100 may rotate the reaction device 20. When a turntable that transmits the rotational force provided by the test apparatus 100 is inserted into a center hole C formed at the center of the reaction device 20 and then the reaction device 20 is rotated, the sample introduced through the inlet hole 21a may move to the chamber 22a by the centrifugal force . When the sample is blood, centrifugal separation by rotation is allowed. Therefore, structures for centrifugal separation of blood may be further provided on the platform 21.

As for the disk type reaction device 20, the platform 21 may include a plate having a plurality of layers. For example, when the platform 21 includes two plates, i.e., an upper plate and a lower plate, an intaglio structure corresponding to the structure, such as a chamber or channel, is formed on a surface on which the upper plate and the lower plate are in contact with each other. Since the two plates are bonded to each other, a space in which a fluid is accommodated and a path on which the fluid moves, may be formed in the platform 21. Bonding of the plates may be performed using one of various methods, such as adhesion using an adhesive or a double-sided adhesive tape, ultrasonic fusion, or laser welding.

On the other hand, the reaction devices 10 and 20 according to the examples of FIGS. 2 and 4 may allow a quantitative analysis by using only a small amount of sample. Since the sample or reagent moves along the channel, the reagent and sample may be in the form of fluid. Therefore, these reaction devices 10 and 20 are referred to as a microfluidic device.

The appearance and the type of the test apparatuses 100 are not limited to the examples shown in FIGS. 1 and 3, an apparatus such as a spectrometer for testing a cuvette-type reaction device may also be the test apparatus 100 according to an embodiment. Therefore, an apparatus capable of performing a test in which a result is affected by an external temperature, an internal temperature or a temperature of a sample may be a test apparatus according to an embodiment.

As described above, the chambers 12a, and 22a may accommodate different reagents, and thus test for various test items may be performed at the same time.

For example, in each of chambers 12a and 22a, a GGT test reagent, a CREA test reagent, a TRIG test reagent, a CHOL test reagent, an ALT test reagent may be accommodated. Therefore, it may be possible to simultaneously perform a GGT test, a CREA test, a TRIG test, a CHOL test, and an ALT test.

A battery 180 is mounted on the test apparatus 100 described in FIGS. 1 and 3, and thus according to the amount of power of the battery, the test apparatus 100 may obtain the electric power required for the test. As will be described later, the battery 180 has various types, and information of the battery 180 related to the power consumption rate may vary depending on the type of the battery 180, wherein the information of the battery 180 may include the power consumption rate based on a manufacturing date of the battery 180 and the number of uses of the battery 180, and information related to a temperature of the battery 180.

When a test is performed on a test medium by the operation of the test apparatus 100, the power of the battery 180 may consume and thus the current amount of power may be determined according to the power consumption of the battery 180. In other words, a period of time in which the test apparatus 100 is operable for the test process and the number of test times may vary according to the current amount of power of the battery 180, and thus it may be important for a user to recognize the current amount of power of the battery 180.

According to a conventional manner, a test apparatus 100 identifies the amount of power of a battery 180 based on data related to a predetermined power consumption rate of the battery 180, instead of calculating the power consumption rate of the battery 180. That is, when the test apparatus 100 proceeds with the test operation, the test apparatus 100 may uniformly identify the power consumption of the battery 180 based on the number of the test operation and the elapsed time.

However, the battery 180 has a different power consumption rate depending on its type, and the power consumption rate of the battery 180 may vary according to the manufacturing date, the number of uses of the battery 180, the type of test performed by the test apparatus 100, and the temperature of the battery 180. Therefore, when the test of the test apparatus 100 is performed, it is needed to calculate the power consumption rate of the battery 180 based on information related to the battery 180.

According to the conventional manner, the test apparatus 100 displays the current amount of power of the battery 180 to inform a user of the current amount of power of the battery 180 when the current amount of power of the battery 180 is identified. Accordingly, the user may recognize the displayed current amount of power of the battery 180, but the user does not recognize the period of time in which the test apparatus 100 is operable for the test or the available number of test times that are changed according to the power consumption of the battery 180. Therefore, the test may be terminated due to the lack of power of the test apparatus 100 during the test is performed by the test apparatus 100. As for the portable POC device, when the test is terminated during performing the test in the outside, it is impossible to restart the test, and as for the drug test, it may be impossible to test a test medium in time.

According to an embodiment, the test apparatus and the control method thereof may calculate the variation of the power consumption of the battery 180 according to the performance of the test, based on the information of the battery 180 that is pre-stored. In addition, the test apparatus 100 may display the available operation time and the available number of test times of the battery 180 according to the current amount of power of the battery 180 that is identified based on the calculated power consumption rate of the battery 180. Therefore, a user may prepare the discharge of the battery 180 and when it is impossible to proceed with the test since the current amount of power of the battery 180 is low, the user may terminate the test.

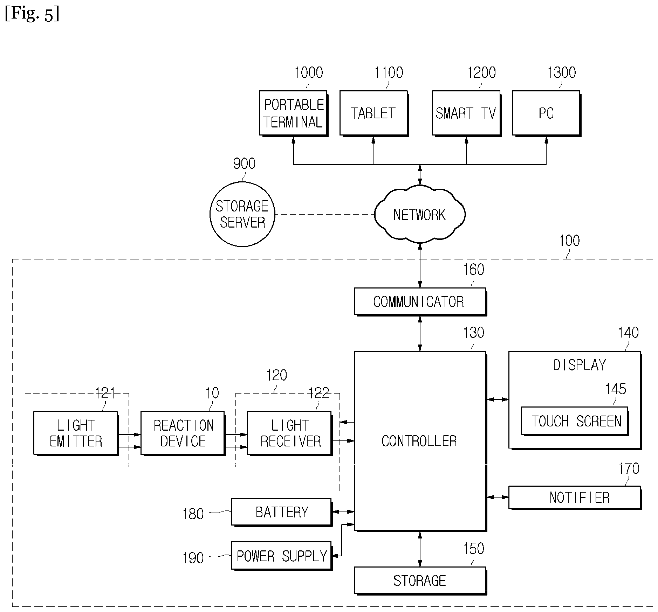

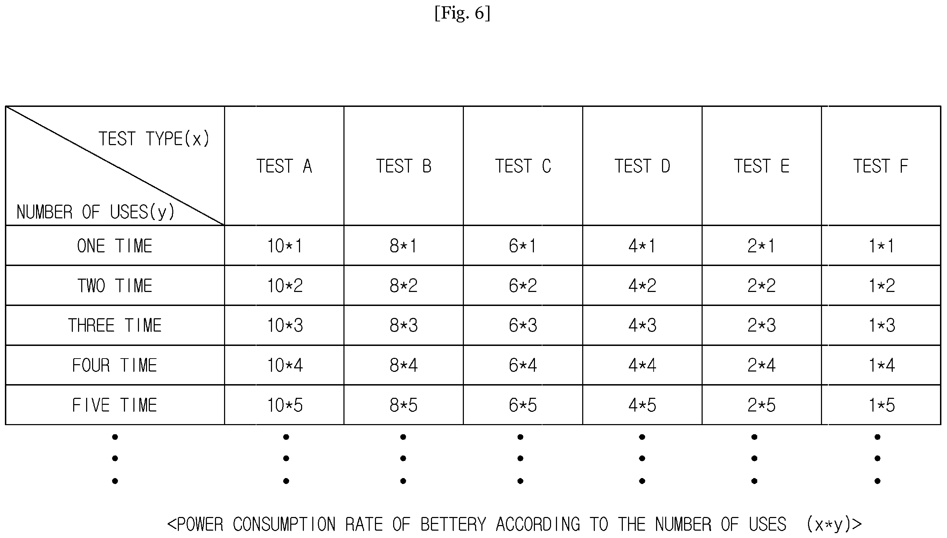

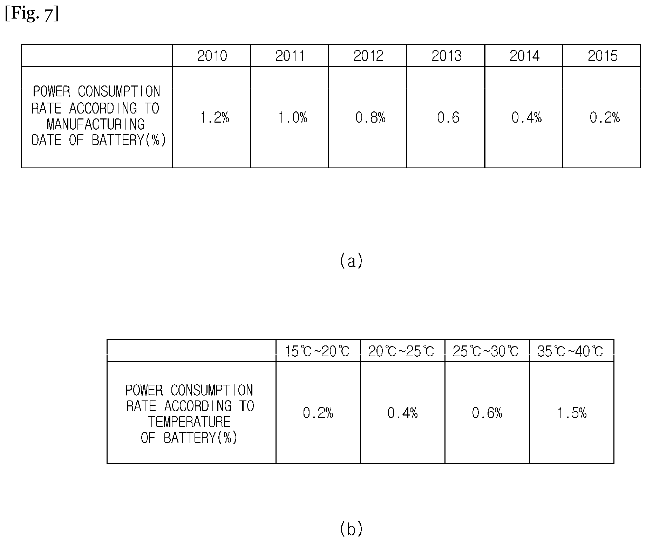

FIG. 5 is a control block diagram illustrating a configuration of the test apparatus according to an embodiment, FIG. 6 illustrates data on a power consumption rate based on the type of test apparatus and the number of uses of a battery according to an embodiment, and FIG. 7 illustrates data on a power consumption rate based on a manufacturing data of the battery and the temperature of the battery according to an embodiment.

Referring to FIG. 5, the test apparatus 100 may include a detector 120 detecting a sample placed in the reaction device 10 by emitting light to the chamber and detecting an optical signal from the chamber, a controller 130 controlling the overall operation of the test apparatus 100, a display 140 providing information related to the operation and control of the test apparatus 100 to a user, a storage 150 storing data related to the control of the test apparatus 100, a communicator 160 allowing the test apparatus 100 to transmit and receive data with an external server and transmitting and receiving data related to the operation and control of the test apparatus 100 to and from an external device, a notifier 170 transmitting information related to the operation and control of the test apparatus 100 to a user as a notification, the battery 180 proving the power to operate the test apparatus 100 and a power supply 190 controlling the power of components of the test apparatus 100 according to the power provided from the battery 180.

As mentioned above, the reaction device 10 is a device in which biochemical reaction takes place to identify the presence of a target object contained in the sample or to calculate a density of a target object by receiving biochemical sample, such as blood. The detector 120 may include a light emitter 121 and a light receiver 122.

The light emitter 121 may be implemented by a planar light source having a great light emission area to uniformly emit light over a certain area of the reaction device 10. For example, the light emitter 121 may be a backlight. In addition, the light emitter 121 may be a light source to flash on and off at a predetermined wave, and may be implemented by any one of a semiconductor light emitter, such as a Light Emitting Diode (LED) or Laser Diode (LD), or a gas discharge lamp, such as a halogen lamp or xenon lamp.

The light receiver 122 may generate an electrical signal according to intensity of light by detecting light which is emitted from the light emitter 121 and then penetrates the sample placed in the chamber 12a or is reflected from the sample placed in the chamber 12a. The light receiver 122 may include depletion layer photo diode, avalanche photo diode or photomultiplier tube. The light receiver 122 may be implemented by CMOS image sensor or CCD image sensor.

The light emitter 121 and the light receiver 122 may be provided to face each other with respect with the reaction device 10, or provided on an upper portion of the reaction device 10 and a lower portion of the reaction device 10, respectively. The light emitter 121 and the light receiver 122 may be moved in a direction, in which the detector 120 is arranged, to detect a result of reaction of the detector 120. A power for the movement of the light emitter 121 and the light receiver 122 may be supplied from the motor (not shown) of the test apparatus 100. The controller 130 may control driving of the motor to control the movement of the light emitter 121 and the light receiver 122.

The intensity and waves of light emitted from the light emitter 121 may be controlled by a command from the controller 130. The light receiver 122 may send an electrical signal generated by detecting light, to the controller 130. The light emitter 121 and the light receiver 122 may further include an AD converter configured to convert a detection result of the light receiver 122 into a digital signal to output the digital signal to the controller 130.

The controller 130 may control the operation related to the control method of the test apparatus 100 according to an embodiment. Particularly, the controller 130 may calculate the power consumption rate of the battery 180 that is changed according to the operation of the test apparatus 100 based on the information of the battery 180 stored in the storage 150, and determine the current amount of power of the battery 180 based on the calculated power consumption rate of the battery 180.

The information on the battery 180 stored in the storage 150 may include at least one of the power consumption rate based on a manufacturing date of the battery 180, the number of uses of the battery 180, a power consumption rate based on the number of uses of the battery 180, a power consumption rate based on the temperature of the battery 180, and a power consumption rate based on the type of test that the test apparatus 100 performs.

The battery 180 provided in the test apparatus 100 may transmit information on the power consumption rate to the controller 130. In addition, since the power consumption rate varies according to the information of the battery 180 as described above, there are various cases where the power consumption rate of the battery 180 is changed when the test apparatus 100 proceeds with a test.

Referring to FIG. 7A, the power consumption rate of the battery 180 may vary according to the manufacturing date. That is, when the manufacturing date of the battery 180 is old, it may lead the degradation of the battery 180 and thus when the test apparatus 100 proceeds with the test, the power consumption of the battery 180 may be increased. Further, even when the test apparatus 100 does not proceed with the test, the power of the battery 180 may be consumed, wherein the consumption of the battery 180 is different according to the manufacturing date of the battery 180.

As shown in FIG. 7A, when the year of manufacture of the battery 180 is 2010, the power consumption rate of the battery 180 is 1.2%. When the year of manufacture of the battery 180 is 2015, the power consumption rate of the battery 180 is 0.2%. That is, when the year of manufacture of the battery 180 is long with respect to the current time, the performance of the battery 180 may be decreased accordingly and thus the power consumption of the battery 180 is greater than when the year of manufacture of the battery 180 is not for a long time.

Referring to FIG. 6, the power consumption rate of the battery 180, which is based on the type of test performed by the test apparatus 100, may vary depending on the type of test performed by the test apparatus 100. That is, the types of test performed by the test apparatus 100 may include a blood test, a heart disease test, a drug use test, and an AIDS test, and thus the operation time and the configuration of the battery 180 that is operated may vary depending on the type of test. Therefore, the power consumption rate of the battery 180 may be changed depending on the type of test.

In addition, when the test apparatus 100 performs the test for a plurality of times, the performance of the battery 180 may be continuously decreased and thus the power consumption of the battery 180 may be increased according to the number of uses.

For example, as shown in FIG. 6, when the test apparatus 100 drives the driving motor (not shown) for 15 minutes, to perform a test A, the power consumption rate of the battery 180 may be 10%. When the test apparatus 100 drives the driving motor (not shown) for 10 minutes, to perform a test C, the power consumption rate of the battery 180 may be 6%.

When the test apparatus 100 proceeds with the test, the controller 130 may calculate the power consumption rate of the battery 180 mounted to the test apparatus 100 based on the information of the battery 180 stored in the storage 150, wherein the calculation is performed by using equation 1.

[116]    Equation 1 $$c=d+x*y$$

c is the power consumption rate of the battery 180, d is the power consumption rate of the battery 180 based on the year of manufacture of the battery 180, x is the power consumption rate depending on the type of test performed by the test apparatus 100, y is the number of uses according to the test process, and a unit of the power consumption rate of the battery 180 is expressed as a percentage (%).

For example, referring to FIGS. 6 and 7, when the test apparatus 100, to which the battery 180 manufactured in 2012, proceeds with the test A, the controller 130 may calculate the power consumption rate of the battery 180 based on the information of the battery 180 stored in the storage 150.

The power consumption of the battery 180 takes place even when the test A is not performed. Therefore, when the number of uses of the battery 180 is 0 (zero), the power consumption rate of the battery 180 may be 0.8%. Since the power consumption rate of the battery 180 is calculated as c = 0.8 + 10 * 1 when the test apparatus 100 proceeds with the test A for one time, the power of the battery 180 may be consumed by 10.8%. Therefore, it is assumed that the amount of power of the battery 180 is 100% when the power of the battery 180 is not consumed, and thus when the test apparatus 100 proceeds with the test A for one time, the amount of power of the battery 180 becomes 89. 2 %.

When the test apparatus 100 proceeds with the test A for two times, the power consumption rate of the battery 180 is calculated as c = 0.8 + 10 * 2, and thus the power of the battery 180 may be consumed by 20.8%. Therefore, when the test apparatus 100 proceeds with the test A for two times, the amount of power of the battery 180 becomes 68. 4 %.

As mentioned above, when the test apparatus 100 proceeds with the test, the controller 130 may calculate the power consumption rate of the battery 180 based on the type of test and the information of the battery 180, and determine the current amount of power of the battery 180 based on the calculated power consumption rate.

As illustrated in FIG. 7B, the power consumption rate of the battery 180 may vary depending on the temperature of the battery 180. An optimum operating temperature for normally supplying power to the battery 180 provided in the test apparatus 100 is 15 °C to 32 °C. In addition, the power consumption rate of the battery 180 is low at the low temperature and high at the high temperature.

Referring to FIG. 7B, when the temperature of the battery 180 is 15 °C to 20 °C, the power consumption rate of the battery 180 is 0.2%, and when the temperature of the battery 180 is 20 °C to 25 °C, the power consumption rate of the battery 180 is 0.4%. When the temperature of the battery 180 is 25 °C to 30 °C, the power consumption rate of the battery 180 is 0.6%, and when the temperature of the battery 180 is 30 °C to 40 °C, the power consumption rate of the battery 180 is 1.5%. That is, when the temperature of the battery 180 is not in the optimum operating temperature, the power consumption rate may be further increased.

The above mentioned equation 1 and figures are merely the embodiments of the disclosure, and thus equations and figures for implementing the control method of the test apparatus according to an embodiment may vary.

The controller 130 may determine whether the test apparatus 100 proceeds with the test based on the current amount of power of the battery 180 determined as described above. That is, since the needed power of the battery 180 is different depending on the type of the test that can be performed by the test apparatus 100, the controller 130 may calculate the amount of power of the battery 180 depending on the type of the test set by the user or the type of the test pre-stored in the storage 150.

The controller 130 may compare the amount of power of the battery 180 that is needed for the test performed by the test apparatus 100, with the calculated current amount of power of the battery 180, and when the current amount of power is greater than the amount of power of the battery 180 for the test, the controller 130 may transmit a control signal for proceeding the test. That is, when the test apparatus 100 is capable of performing the test for one or more times since the current amount of power of the battery 180, which is calculated by the controller 130, exceeds a predetermined amount of power, the controller 130 may transmit a control signal for proceeding with the test.

Further, the controller 130 may control the power needed for driving of the test apparatus 100, by determining the current amount of power of the battery 180. That is, when the current amount of power of the battery 180 is equal to or less than the predetermined amount of power, the controller 130 may transmit a control signal for reducing the power consumption of the test apparatus 100. Particularly, when the test apparatus 100 does not proceed with the test, the controller 130 may reduce the power consumption by stopping the operation of the heater, the fan, the motor, and the bio-sensor, which are involved in the test process, among the configuration of the test apparatus 100.

When the controller 130 determines that the test apparatus 100 cannot perform the test, which is to be performed by the test apparatus 100, even for one time since the current amount of power of the battery 180 is equal to or less than the predetermined amount of power, the controller 130 may control the power supply 190 to prevent the test from being proceeded by cutting off the power that is supplied to the configuration involved in the test process from the battery 180. Accordingly, even when the current amount of power of the battery 180 is small, it may be possible to prevent a case where the test is stopped during a user proceeds with the test.

The controller 130 may transmit a control signal to the display 140 so that the display 140 displays the current amount of power of the battery 180 and at least one piece of information related to the operation of the test apparatus 100, which is described later. In addition, when the current amount of power of the battery 180 is equal to or less than the predetermined amount of power, the controller 130 may transmit a control signal to the display 140 so that the display 140 does not display a control screen configured to perform the test process of the test apparatus 100 or the controller 130 may transmit a control signal so that the notifier 170 informs a user of the lack of the current amount of power of the battery 180.

The controller 130 may be embedded in the test apparatus 100. The controller 130 may include a main processor, a graphic processor and a memory.

The display 140 may display a result of the test performed by the test apparatus 100. As mentioned above, the reaction device 10 includes a plurality of chambers 12a, and thus a single reaction device 10 detects a large number of test items. When the plurality of test items is detected, the display 140 may display a result of the plurality of test items. Further, the display 140 may provide the information related to the test apparatus 100 to a user. For example, the display 140 may provide setting of the test apparatus 100, and the progress state of the test, the test results, to the user and may indicate whether the test of the test apparatus 100 is proceeded or not, based on the current amount of power of the battery 180.

The display 140 may be implemented by a Liquid Crystal Display (LCD), a Light Emitting Diodes (LED) display, an Organic Light Emitting Diodes (OLED) display, an Active Matrix Organic Light Emitting Diodes (AMOLED) display, a flexible display, and a three-dimensional display (3D display). In addition, the display 140 may include a touch screen 145 receiving a touch command from a user. Hereinafter, for convenience of description, the display 140 of the test apparatus 100 is implemented by the touch screen 145 as an example.

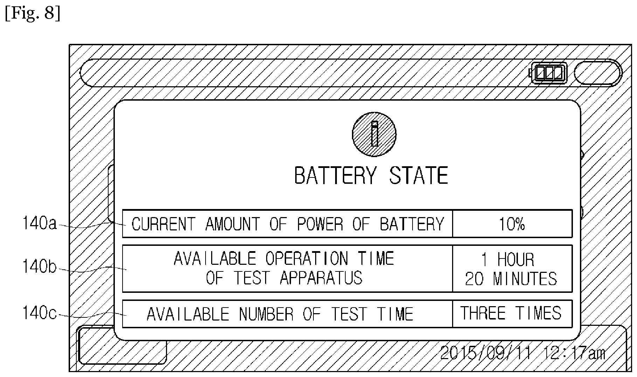

In addition, the display 140 may include a screen implemented by the control method of the test apparatus 100 according to an embodiment. Particularly, the display 140 may display the current amount of power that is determined by the controller 130 and at least one piece of information related to the operation of the test apparatus 100, and a description thereof will be described with reference to FIGS. 8 to 12.

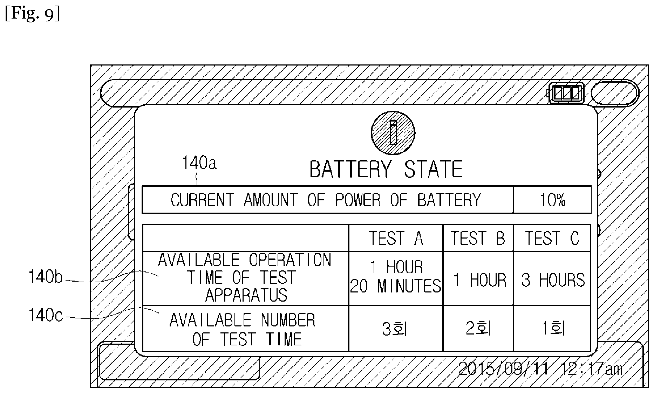

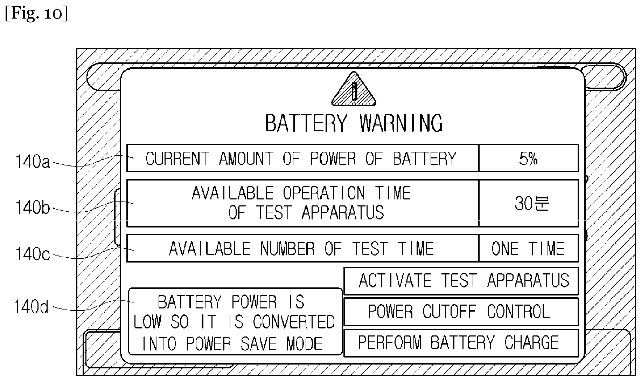

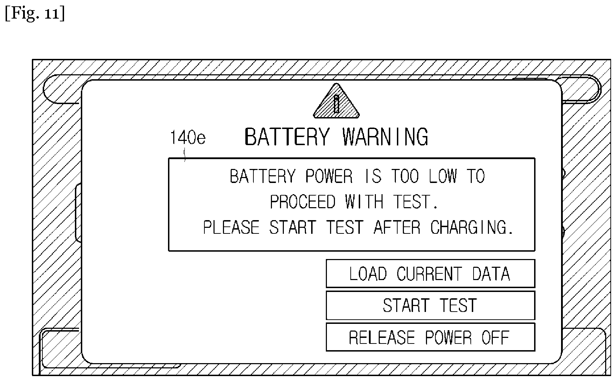

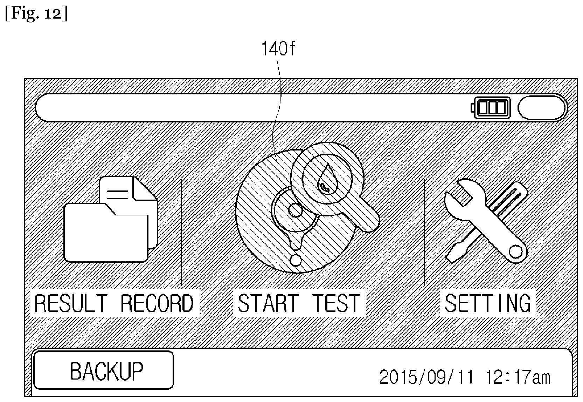

FIG. 8 illustrates displaying of information related to a current power amount of the battery and the operation of the test apparatus according to an embodiment, and FIG. 9 illustrates displaying of information related to a current power amount of a battery and the operation of a test apparatus according another embodiment. FIG. 10 is a screen illustrating a control of a power supply for driving the test apparatus according to an embodiment, FIG. 11 is a screen illustrating that the power supplied for the test process of the test apparatus is cut off, and FIG. 12 is a screen illustrating a control screen for the test process of the test apparatus.

Referring to FIG. 8, the display 140 may display the current amount of power 140a of the battery, the available operation time 140b indicating an available period of time in which the test apparatus 100 is operable to proceed with the test, and an available number of test times 140c in which the test apparatus 100 can proceed with the test. The number of items that can be displayed on the display 140 are not limited, and various embodiments may be allowed.

As shown in FIG. 8, when the current amount of power 140a of the battery 180, which is determined based on the power consumption rate of the battery 180 calculated by the controller 130, is 10%, the available operation time 140b in which the test apparatus 100 is operable by using the amount of power of 10% may be 1 hour and 20 minutes, and the available number of test times 140c in which the test apparatus 100 can proceed with the test by using the amount of power of 10% may be three times. Accordingly, a user may recognize the current amount of power 140a of the battery 180, the available operation time 140b of the test apparatus 100, and the available number of test times 140c of the test apparatus 100 displayed on the display 140 and the user may determine whether to proceed with the test using the test apparatus 100.

Referring to FIG.9, the display 140 may display the current amount of power 140a of the battery 180 and at the same time, the display 140 may display the available operation time 140b of the test apparatus 100 and the available number of test times 140c of the test apparatus 100 depending on the type of test proceeded by the test apparatus 100.

As illustrated in FIG. 6, the power consumption rate of the battery 180, which is based on the type of test performed by the test apparatus 100, may vary depending on the type of test performed by the test apparatus 100. Further, the operation time and the configuration of the battery 180 that is operated may vary depending on the type of test, and thus the consumed amount of power of the battery 180 may be different although based on the same amount of power of the battery 180. Therefore, although based on the same amount of power, the available operation time in which the test apparatus 100 is operable or the available number of test times in which the test is proceeded may vary depending on the type of test

As shown in FIG. 9, when the current amount of power140a of the battery 180 displayed on the display 140 is 10%, the available operation time 140b of the test apparatus 100 for the test A may be 1 hour and 20 minutes, and the available number of test times 140c for the test A is three times. In addition, an available operation time for the test B is 1 hour and an available number of test times for the test B is two times. An available operation time for the test C is 3 hours and an available number of test times for the test C is one time.

A user may recognize the available operation time and the available number of test times of the test apparatus 100 depending on the type of test, through a screen displayed on the display 140. FIG. 9 illustrates the tests A, B and C as an example, but the type of test and the number of test is not limited thereto. Therefore, a variety of embodiment may be provided.

Referring to FIG. 10, the display 140 may display a control screen for the progress of the test of the test apparatus 100 based on the current amount of power of the battery 180. The controller 130 may determine the current amount of power of the battery 180, and compare the determined current amount of power with the predetermined current amount of power. As a result of the comparison, when it is needed to save the power since the determined current amount of power is equal to or less than the predetermined current amount of power, the controller 130 may control the power needed for driving of the test apparatus 100. That is, the controller 130 may transmit a control signal for reducing the power consumption, so as to stop the operation of the heater, the fan, the motor, and the bio-sensor, thereby reducing the power consumption.

The display 140 may display a notification notifying that the controller 130 controls the power, on the screen. As illustrated in FIG. 10, when the current amount of power of the battery 180 is 5%, the available operation time of the test apparatus 100 is 30 minutes, and the available number of test time is one time, it may be needed to save the power consumed in the test apparatus 100. Therefore, the controller 130 may transmit a control signal for controlling the power and at the same time, the display 140 may display a notification screen 140d notifying that the test apparatus 100 is converted into a power save mode. Therefore, the test apparatus 100 may save the power for proceeding with the test and a user may recognize that the test apparatus 100 is converted into the power save mode.

A user may input a control command for the test apparatus 100 by touching the touch screen 145 provided in the display 140. Particularly, the user may input a control command for re-inactivating the test apparatus 100 in the power save mode, a control command for stopping the test apparatus 100 by cutting off the power of the test apparatus 100, and a control command for charging the battery 180.

Referring to FIG. 11, the display 140 may display a control screen for stopping the test process of the test apparatus 100. That is, when the test apparatus 100 is not allowed to proceed with the test since the current amount of power of the battery 180 determined by the controller 130 is less than the predetermined amount of power, the display 140 may display a screen 140e notifying that it is impossible to proceed with the test. A user may recognize that the current amount of power of the battery 180 is too low to perform the test, through the screen displayed on the display 140, and the user may not allow the test apparatus 100 to proceed with the test, thereby preventing a case where the operation of the test apparatus 100 is stopped during the test process.

A user may load data related to the test currently performed, by touching the touch screen 145, wherein the data is stored in the test apparatus 100. The user may perform a touch input to start the test after connecting a charging cable for charging the battery 180 and the user may input a power off release command for releasing a mode in which the power of the test apparatus 100 is cut off.

Referring to FIG. 12, the display 140 may display a test control screen, which is inactivated, so as to prevent the test apparatus 100 from proceeding with the test. In order that the test apparatus 100 starts to proceed with the test on the test medium, a user may input a control command for starting the test by touching the touch screen 145 of the display 140. That is, as illustrated in FIG. 12, the test on the test medium may be proceeded when a user presses a test start button 140f displayed on the touch screen 145.

However, in this case, when a user proceeds with the test by pressing the test start button 140f although the power capacity of the battery 180 is too small to perform the test, the test process may be terminated in the middle of the test since the operation of the test apparatus 100 is stopped.

Therefore, as mentioned above, when the test apparatus 100 cannot perform the test even for one time since the current amount of power of the battery 180 is equal to or less than the predetermined amount of power, the controller 130 may transmit the control signal for cutting off the power of the test apparatus 100 and at the same time, the controller 130 may prevent the user from inputting the control command for proceeding with the test, by inactivating the test start button 140f displayed on the display 140.

The notifier 170 may transmit a notification to the user when the current amount of power of the battery 180 determined by the controller 130 is equal to or less than the predetermined amount of power. That is, when the current amount of power of the battery 180 is too low and thus the test apparatus 100 cannot proceed with the test, the notifier 170 may notify a user that the current amount of power of the battery 180 is insufficient through a voice signal or a mechanical sound. The notifier 170 may be provided in the form of a general speaker or a diaphragm capable of outputting a sound only. The notifier 170 may be separately installed on the outside of the test apparatus 100 or installed in the test apparatus 100.

The storage 150 may store data related to the operation and control of the test apparatus 100. Particularly, the storage 150 may store at least one of the power consumption rate based on the manufacturing date, the number of uses of the battery 180, the power consumption rate based on the number of uses of the battery 180, the power consumption rate based on the temperature of the battery 180, and the power consumption rate based on the type of test performed by the test apparatus. The storage 150 may store information related to the amount of power of the battery 180, which is for the test apparatus 100 to proceed with the test, a reference amount of power of the battery 180, which is needed for the test apparatus 100 to proceed with the test depending on the type of test, and a reference amount of power of the battery 180, which is for inactivating the test start button 140f to prevent the test from being conducted.

In addition, the storage 150 may store information related to the power consumption rate of the battery 180 calculated by the controller 130, and information related to the current amount of power of the battery 180 determined based on the calculated power consumption rate. The storage 150 may provide the accumulated information to the controller 130 so that the information may be used to calculate a power consumption rate of the battery 180 and a current power amount of the battery 180 in further.

The storage 150 may include a high-speed random access memory, a magnetic disk, an S-RAM, a dynamic random access memory (DRAM), or a read only memory (ROM) but is not limited thereto. In addition, the storage 150 may be detachably attached to the test apparatus 100. For example, the storage 150 may include a Compact Flash (CF) Card, a Secure Digital (SD) Card, a Smart Media (SM) card, a Multimedia Card (MMC) or a Memory Stick, but is not limited thereto.

The communicator 160 may perform wired / wireless communication between the test apparatus 100 and an external device. Particularly, the communicator 160 may transmit data that is obtained by the test apparatus 100 or stored in the test apparatus 100, to the external device so that contents displayed on the display 140 are displayed on the external device.

As illustrated in FIGS. 8 to 12, according to an embodiment, data related to the screen displayed on the display 140 of the test apparatus 100 may be transmitted to the external device and thus a user may intuitively recognize the screen displayed on the test apparatus 100 through the external device.

The communicator 160 may transmit the control signal for allowing the current amount of power of the battery 180 and at least one of the information related to the operation of the test apparatus 100 to be displayed on the external device based on the current amount of power of the battery 180 determined by the controller 130.

The external device may include a portable terminal 1000, a tablet 1100, a smart TV 1200 and a PC 1300, but is not limited thereto. The external device may include an apparatus having a display displaying a screen that is the same as a screen output from the display 140 of the test apparatus 100, by being wired or wirelessly connected to the test apparatus 100. Further, the communicator 160 may be connected a storage server 900 via the network, and when the controller 130 loads data stored in the storage server 900, the communicator 160 may transmit the data to the controller.

The communicator 160 may include at least one of a Bluetooth communication module communicating with a single external device in one to one manner, or communicating with a small number of external device in one to many manner, a wireless fidelity (WiFi) communication module being connected to local area network (LAN) via an access point, and a near field communication module such as Zigbee communication module generating a near field communication network between the test apparatus 100 and the external device.

However, the communication module contained in the communicator 160 is not limited to the Bluetooth communication module, the WiFi communication module and the near field communication module. Therefore, the communicator 160 may include a variety of communication modules performing the communication according to a variety of communication protocols.

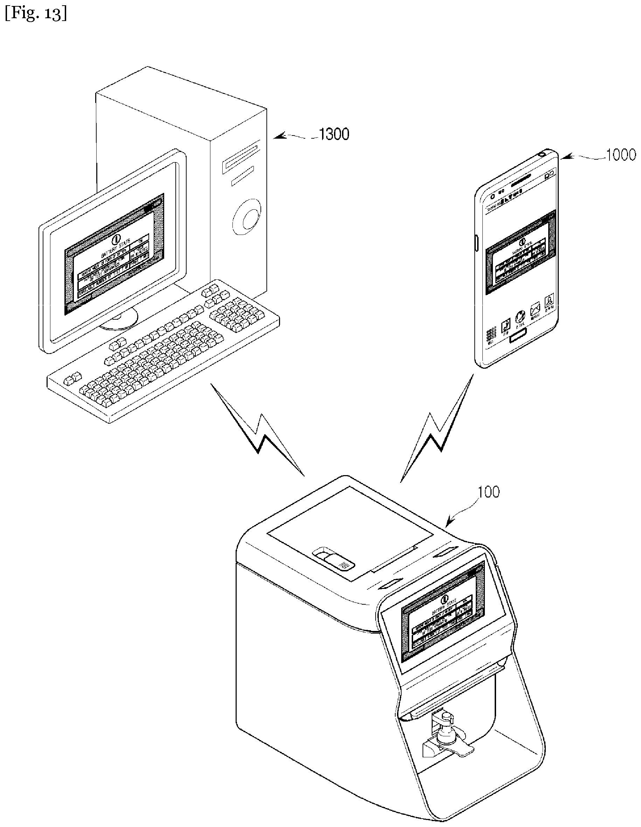

FIG. 13 is a view illustrating that the amount of power of the battery and at least one piece of information related to the operation of the test apparatus is displayed on an external device according to an embodiment.

As mentioned above, as illustrated in FIGS. 8 to 12, according to an embodiment, data related to the screen displayed on the display 140 of the test apparatus 100 may be transmitted to the external device and thus a user may intuitively recognize the screen displayed on the test apparatus 100 through the external device. As illustrated in FIG.

13, the current amount of power 140a of the battery, the available operation time 140b indicating an available period of time in which the test apparatus 100 is operable to proceed with the test, and the available number of test times 140c in which the test apparatus 100 proceeds with the test, may be displayed on a screen of the portable terminal 1000 or the PC 1300 corresponding to the external device.

In addition, the external device may output a notification to a user in the same manner as the notifier 170 of the test apparatus 100. That is, when the current amount of power of the battery 180 is too low and thus the test apparatus 100 cannot proceed with the test, the notifier 170 may notify a user that the current amount of power of the battery 180 is insufficient through a voice signal or a mechanical sound.

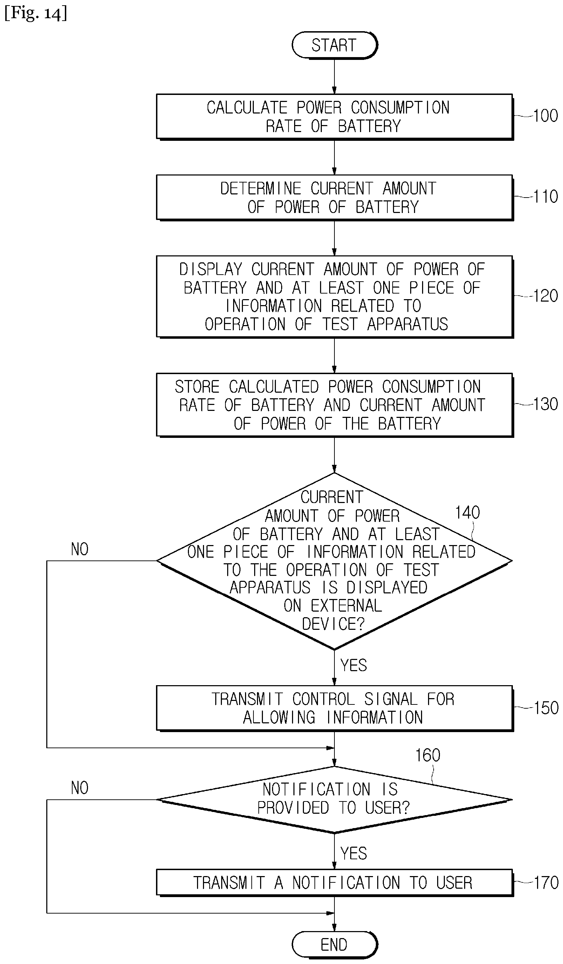

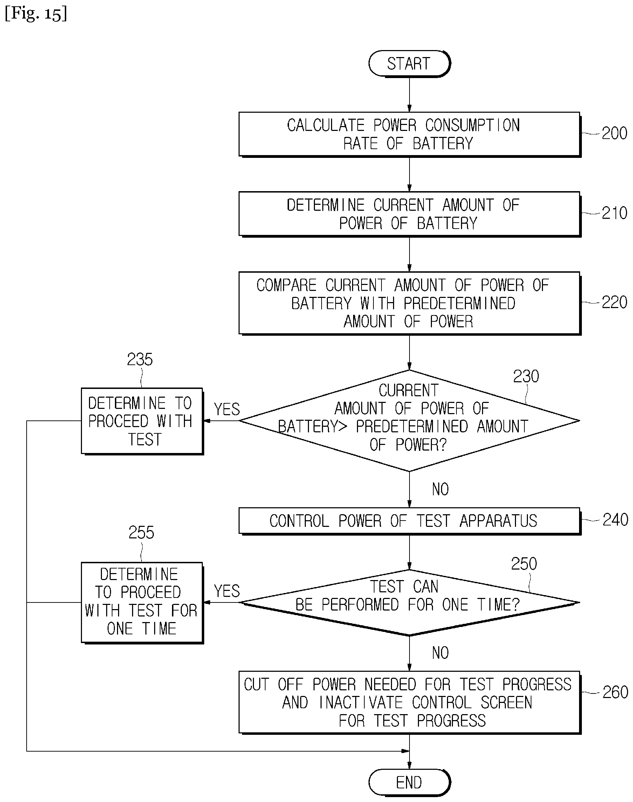

FIG. 14 is a flowchart illustrating a method of controlling the test apparatus according to an embodiment, and FIG. 15 is a flowchart illustrating a method of controlling a test apparatus according to another embodiment.

Referring to FIG. 14, the controller 130 may calculate the power consumption rate of the battery 180 mounted to the test apparatus 100 (100). The storage 150 may store the information of the battery 180 such as the power consumption rate based on the manufacturing date, the number of uses of the battery 180, the power consumption rate based on the number of uses of the battery 180, the power consumption rate based on the temperature of the battery 180, and the power consumption rate based on the type of test performed by the test apparatus, and thus the controller 130 may calculate the power consumption rate of the battery 180, which is changed according to the operation of the controller 130, based on the information of the battery 180.

The controller 130 may calculate the power consumption rate of the battery 180 and subtract the consumed amount of power from the amount of power of the battery 180, which is before calculating the power consumption rate, thereby determining the current amount of power of the battery 180 (110).

When the current amount of power of the battery 180 is determined, the display 140 may display the current amount of power of the battery 180 and at least one piece of information related to the operation of the test apparatus 100, under the control of the controller 130 (120). That is, the display 140 may display the current amount of power of the battery 180, the available operation time of the test apparatus 100 and the available number of test times. A user may recognize the current amount of power of the battery 180, and information, which is needed for the test apparatus 100 to proceed with the test, based on the information displayed on the display 140.

The controller 130 may store the power consumption rate of the battery 180 and the current amount of power of the battery 180 determined based on the calculated power consumption rate, in the storage 150 (130). The accumulated information may be provided to the controller 130 so that the information may be used to calculate the power consumption rate of the battery 180 and the current power amount of the battery 180.

In addition, the controller 130 may determine whether the test apparatus 100 is connected to the external device via the communicator 160, and when the test apparatus 100 is connected to the external device via the communicator 160, the controller 130 may determine whether to display the current amount of power of the battery 180 and at least one piece of information related to the operation of the test apparatus 100, on the external device, or not (140). The controller 130 may determine whether to display the information on the external device, based on setting data that is set by a user in advance.

When the controller 130 determines to display the information on the external device, the controller 130 may control the communicator 160 so that the communicator 160 transmit the control signal for allowing the above mentioned information to be displayed on the external device (150).

The controller 130 may determine to notify a user of a notification when the current amount of power of the battery 180 is equal to or less than the predetermined amount of power (160) and may transmit the control signal to the notifier 170. The notifier 170 may transmit a notification to a user based on the control signal received from the controller 130 (170), and when the current amount of power of the battery 180 is too low and thus the test apparatus 100 cannot proceed with the test, the notifier 170 may notify a user that the current amount of power of the battery 180 is insufficient through a voice signal or a mechanical sound.

Referring to FIG. 15, the controller 130 may calculate the power consumption rate of the battery 180 mounted to the test apparatus 100 (200). In the storage 150, the power consumption rate based on the manufacturing date of the battery 180, the number of uses of the battery 180, the power consumption rate based on the number of uses of the battery 180, the power consumption rate based on the temperature of the battery 180, and the power consumption rate based on the type of test performed by the test apparatus may be stored. Based on the information of the battery 180, the controller 130 may calculate the power consumption rate of the battery 180 that is changed according to the operation of the test apparatus 100.

The controller 130 may calculate the power consumption rate of the battery 180 and subtract the consumed amount of power from the amount of power of the battery 180, which is before calculating the power consumption rate, thereby determining the current amount of power of the battery 180 (210). The controller 130 may calculate the power consumption rate of the battery 180 based on the type of test the test apparatus 100 can perform.

The controller 130 may compare the current amount of power of the battery 180 with the pre-determined amount of power of the battery 180 stored in the storage 150 (220). As a result of the comparison, when the current amount of power of the battery 180 is greater than the predetermined amount of power, the controller 130 may determine that the test apparatus 100 can proceed with the test (235). In contrast, when the determined amount of power of the battery 180 is equal to or less than the predetermined amount of power, the controller 130 may transmit a control signal for controlling the power for the drive of the test apparatus 100.

That is, when it is needed to reduce the power consumption of the test apparatus 100 since the determined current amount of power is equal to or less than the predetermined current amount of power, the controller 130 may stop the operation of the heater, the fan, the motor, and the bio-sensor, which are involved in the test process, among the components of the test apparatus 100, thereby reducing the power consumption when the test process is not performed.

The controller 130 may identify whether the test apparatus 100 can proceed with the test for one or more times, based on the current amount of power of the battery 180 (250). As a result of determination, when it is identified that the test apparatus 100 can proceed with for one or more times, the controller 130 may determine to process with the test for one time (255) so as to allow the test apparatus 100 to proceed with test according to the test control command of the user.

When the controller 130 may determine that the test apparatus 100 cannot proceed with the test for one or more times since the determined current amount of power is equal to or less than the predetermined current amount of power, the controller 130 may control the power supply 190 so that the test is not proceeded by preventing the power from being supplied to the components involved in the test process, from the battery 180. In addition, the controller 130 may allow the test control screen displayed on the display 140 to be inactivated (260).

**Claims**

1. A test apparatus configured to test a test medium accommodated in a reaction device, the test apparatus comprising:

   a storage configured to store information related to a battery provided in the test apparatus;
   a controller configured to calculate a power consumption rate of the battery changed according to an operation of the test apparatus based on the stored information of the battery, and configured to determine a current amount of power of the battery based on the calculated power consumption rate of the battery; and
   a display configured to display the current amount of power of the battery and at least one piece of information related to the operation of the test apparatus based on the determined current amount of power of the battery.

2. The test apparatus of claim 1, wherein
   the information related to the operation of the test apparatus comprises at least one of an available operation time and an available number of test times of the test apparatus based on the determined current amount of power of the battery.

3. The test apparatus of claim 1, wherein
   the information of the battery comprises at least one of a power consumption rate based on a manufacturing date of the battery, the number of uses of the battery, a power consumption rate based on the number of uses of the battery, a power consumption rate based on a temperature of the battery, and a power consumption rate based on the type of a test.

4. The test apparatus of claim 1, wherein
   the controller calculates the power consumption rate of the battery changed according to an operation of the test apparatus based on the test type of the test apparatus .

5. The test apparatus of claim 1, wherein
   the controller determines whether to proceed with the test of the test apparatus based on the determined current amount of power of the battery.

6. The test apparatus of claim 1, wherein
   the controller controls the power needed for driving the test apparatus based on the determined current amount of power of the battery.

7. The test apparatus of claim 1, wherein
   when the determined current amount of power of the battery is equal to or less than a predetermined amount of power, the controller transmits a control signal configured to reduce the power consumption of the test apparatus.

8. The test apparatus of claim 1, wherein
when the determined current amount of power of the battery is equal to or less than a predetermined amount of power, the controller cuts off the power needed for a test progress of the test apparatus.

9. The test apparatus of claim 1, wherein
when the determined current amount of power of the battery is equal to or less than a predetermined amount of power, the controller transmits a control signal configured to prevent a control screen for the test progress of the test apparatus from being displayed.

10. The test apparatus of claim 1, wherein
the display displays at least one of an available operation time and an available number of test times of the test apparatus based on the determined current amount of power of the battery.

11. The test apparatus of claim 1, wherein
the display displays at least one of an available operation time and an available number of test times of the test apparatus based on the test type of the test apparatus.

12. The test apparatus of claim 1, wherein
the display displays whether the test apparatus proceeds with the test, based on the determined current amount of power of the battery.

13. The test apparatus of claim 1, wherein
when the determined current amount of power of the battery is equal to or less than a predetermined amount of power, the display does not display a control screen for the test progress of the test apparatus .

14. The test apparatus of claim 1, wherein
the storage stores at least one of a power consumption rate based on a manufacturing date of the battery, the number of uses of the battery, a power consumption rate based on the number of uses of the battery, a power consumption rate based on a temperature of the battery, and a power consumption rate based on the type of test.

15. 16. The test apparatus of claim 1, wherein
the storage stores at least one of the calculated power consumption rate of the battery and the current amount of power of the battery .

[Fig. 1]

[Fig. 2]

[Fig. 3]

<u>100</u>

[Fig. 4]

[Fig. 5]

[Fig. 6]

| TEST TYPE(x)<br><br>NUMBER OF USES(y) | TEST A | TEST B | TEST C | TEST D | TEST E | TEST F |
|---|---|---|---|---|---|---|
| ONE TIME | 10*1 | 8*1 | 6*1 | 4*1 | 2*1 | 1*1 |
| TWO TIME | 10*2 | 8*2 | 6*2 | 4*2 | 2*2 | 1*2 |
| THREE TIME | 10*3 | 8*3 | 6*3 | 4*3 | 2*3 | 1*3 |
| FOUR TIME | 10*4 | 8*4 | 6*4 | 4*4 | 2*4 | 1*4 |
| FIVE TIME | 10*5 | 8*5 | 6*5 | 4*5 | 2*5 | 1*5 |
| ⋮ | ⋮ | ⋮ | ⋮ | ⋮ | ⋮ | ⋮ |

<POWER CONSUMPTION RATE OF BETTERY ACCORDING TO THE NUMBER OF USES (x*y)>

EP 3 385 738 A1

[Fig. 7]

|  | 2010 | 2011 | 2012 | 2013 | 2014 | 2015 |
|---|---|---|---|---|---|---|
| POWER CONSUMPTION RATE ACCORDING TO MANUFACTURING DATE OF BATTERY(%) | 1.2% | 1.0% | 0.8% | 0.6 | 0.4% | 0.2% |

(a)

|  | 15℃~20℃ | 20℃~25℃ | 25℃~30℃ | 35℃~40℃ |
|---|---|---|---|---|
| POWER CONSUMPTION RATE ACCORDING TO TEMPERATURE OF BATTERY(%) | 0.2% | 0.4% | 0.6% | 1.5% |

(b)

[Fig. 8]

BATTERY STATE

| | |
|---|---|
| CURRENT AMOUNT OF POWER OF BATTERY | 10% |
| AVAILABLE OPERATION TIME OF TEST APPARATUS | 1 HOUR 20 MINUTES |
| AVAILABLE NUMBER OF TEST TIME | THREE TIMES |

2015/09/11 12:17am

[Fig. 9]

| | | TEST A | TEST B | TEST C |
|---|---|---|---|---|
| BATTERY STATE | | | | |
| CURRENT AMOUNT OF POWER OF BATTERY | | | | 10% |
| AVAILABLE OPERATION TIME OF TEST APPARATUS | | 1 HOUR 20 MINUTES | 1 HOUR | 3 HOURS |
| AVAILABLE NUMBER OF TEST TIME | | 3회 | 2회 | 1회 |

2015/09/11 12:17am

[Fig. 10]

| 140a | CURRENT AMOUNT OF POWER OF BATTERY | 5% |
| 140b | AVAILABLE OPERATION TIME OF TEST APPARATUS | 30분 |
| 140c | AVAILABLE NUMBER OF TEST TIME | ONE TIME |

BATTERY WARNING

ACTIVATE TEST APPARATUS

140d — BATTERY POWER IS LOW SO IT IS CONVERTED INTO POWER SAVE MODE

POWER CUTOFF CONTROL

PERFORM BATTERY CHARGE

[Fig. 11]

140e  BATTERY WARNING

BATTERY POWER IS TOO LOW TO
PROCEED WITH TEST.
PLEASE START TEST AFTER CHARGING.

LOAD CURRENT DATA

START TEST

RELEASE POWER OFF

[Fig. 12]

140f

RESULT RECORD      START TEST      SETTING

BACKUP              2015/09/11  12:17am

[Fig. 13]

[Fig. 14]

```
                          ( START )
                              |
                              v
              +-------------------------------+
              |   CALCULATE POWER CONSUMPTION  |~100
              |       RATE OF BATTERY          |
              +-------------------------------+
                              |
                              v
              +-------------------------------+
              |     DETERMINE CURRENT AMOUNT   |~110
              |       OF POWER OF BATTERY      |
              +-------------------------------+
                              |
                              v
              +-------------------------------+
              |  DISPLAY CURRENT AMOUNT OF POWER OF |
              |  BATTERY AND AT LEAST ONE PIECE OF  |~120
              |      INFORMATION RELATED TO         |
              |   OPERATION OF TEST APPARATUS       |
              +-------------------------------+
                              |
                              v
              +-------------------------------+
              |  STORE CALCULATED POWER CONSUMPTION |
              |   RATE OF BATTERY AND CURRENT AMOUNT|~130
              |     OF POWER OF THE BATTERY         |
              +-------------------------------+
                              |
                              v
                          CURRENT
                       AMOUNT OF POWER
          NO        OF BATTERY AND AT LEAST       140
        <------ ONE PIECE OF INFORMATION RELATED
        |           TO THE OPERATION OF TEST
        |             APPARATUS IS DISPLAYED
        |                 ON EXTERNAL
        |                   DEVICE?
        |                     |
        |                    YES
        |                     v
        |       +-------------------------------+
        |       |    TRANSMIT CONTROL SIGNAL FOR |~150
        |       |       ALLOWING INFORMATION     |
        |       +-------------------------------+
        |                     |
        +-------------------->|
                              v
          NO            NOTIFICATION IS           160
        <------        PROVIDED TO USER?
        |                     |
        |                    YES
        |                     v
        |       +-------------------------------+
        |       |  TRANSMIT A NOTIFICATION TO USER|~170
        |       +-------------------------------+
        |                     |
        +-------------------->|
                              v
                          ( END )
```

[Fig. 15]

START

CALCULATE POWER CONSUMPTION
RATE OF BATTERY ——200

DETERMINE CURRENT AMOUNT OF
POWER OF BATTERY ——210

COMPARE CURRENT AMOUNT OF POWER OF
BATTERY WITH PREDETERMINED
AMOUNT OF POWER ——220

235

DETERMINE TO
PROCEED WITH
TEST

YES ◄— CURRENT
AMOUNT OF POWER OF
BATTERY> PREDETERMINED AMOUNT
OF POWER? 230

NO

CONTROL POWER OF TEST APPARATUS ——240

255

DETERMINE
TO PROCEED
WITH TEST FOR
ONE TIME

YES ◄— TEST CAN
BE PERFORMED FOR ONE TIME? 250

NO

CUT OFF POWER NEEDED FOR TEST PROGRESS
AND INACTIVATE CONTROL SCREEN
FOR TEST PROGRESS ——260

END

## INTERNATIONAL SEARCH REPORT

| International application No. |
| --- |
| **PCT/KR2016/012590** |

| A. | CLASSIFICATION OF SUBJECT MATTER |
| --- | --- |

*G01R 31/36(2006.01)i, G01N 35/08(2006.01)i, G08B 21/18(2006.01)i, A61B 5/145(2006.01)i*

According to International Patent Classification (IPC) or to both national classification and IPC

| B. | FIELDS SEARCHED |
| --- | --- |

Minimum documentation searched (classification system followed by classification symbols)
G01R 31/36; H01M 10/48; A61B 5/151; A61B 5/145; A61B 5/15; A61B 8/00; A61B 5/00; G01N 35/08; G08B 21/18

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: detection device, battery, consumption rate, residual quantity, display

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 5254233 B2 (PANASONIC CORPORATION) 07 August 2013 See paragraphs [32], [118]-[147], [179] and claims 12, 16. | 1-6,8,10,11,14,15 |
| Y | | 7,9,12,13 |
| Y | JP 2005-323845 A (SHIMADZU CORP.) 24 November 2005 See paragraphs [15]-[17] and figures 2, 3. | 7,9,12,13 |
| A | JP 2011-509101 A (BAYER HEALTHCARE LLC.) 24 March 2011 See claims 20-26 and figure 2a. | 1-15 |
| A | JP 2011-101730 A (TOSHIBA CORP. et al.) 26 May 2011 See claims 1-5 and figures 1-13. | 1-15 |
| A | KR 10-2014-0142342 A (LIFESCAN SCOTLAND LIMITED) 11 December 2014 See paragraphs [14]-[24] and figure 2. | 1-15 |

☐ Further documents are listed in the continuation of Box C.    ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 09 FEBRUARY 2017 (09.02.2017) | **10 FEBRUARY 2017 (10.02.2017)** |

| Name and mailing address of the ISA/KR | Authorized officer |
| --- | --- |
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2016/012590**

| Patent document cited in search report | Publication date | Patent family member | Publication date |
|---|---|---|---|
| JP 5254233 B2 | 07/08/2013 | CN 101772322 A | 07/07/2010 |
| | | CN 101772322 B | 18/01/2012 |
| | | EP 2174592 A1 | 14/04/2010 |
| | | EP 2174592 A4 | 16/05/2012 |
| | | EP 2174592 B1 | 22/05/2013 |
| | | US 2011-0098600 A1 | 28/04/2011 |
| | | WO 2009-019888 A1 | 12/02/2009 |
| JP 2005-323845 A | 24/11/2005 | JP 4370974 B2 | 25/11/2009 |
| JP 2011-509101 A | 24/03/2011 | AR 066807 A1 | 16/09/2009 |
| | | BR PI0820583 A2 | 16/06/2015 |
| | | BR PI0820583 A8 | 22/09/2015 |
| | | CA 2707315 A1 | 18/06/2009 |
| | | CA 2707315 C | 18/11/2014 |
| | | CA 2863480 A1 | 18/06/2009 |
| | | CL 2008001600 A1 | 03/11/2008 |
| | | CN 101896116 A | 24/11/2010 |
| | | CN 101896116 B | 18/06/2014 |
| | | CN 104055523 A | 24/09/2014 |
| | | CN 104055523 B | 24/08/2016 |
| | | CN 104062329 A | 24/09/2014 |
| | | EP 2229093 A1 | 22/09/2010 |
| | | HK 1147410 A1 | 14/11/2014 |
| | | HK 1200076 A1 | 31/07/2015 |
| | | HK 1200215 A1 | 31/07/2015 |
| | | JP 2013-258902 A | 26/12/2013 |
| | | JP 2015-029419 A | 12/02/2015 |
| | | JP 2015-073434 A | 16/04/2015 |
| | | JP 5663310 B2 | 04/02/2015 |
| | | JP 5689923 B2 | 25/03/2015 |
| | | KR 10-1493798 B1 | 02/03/2015 |
| | | KR 10-1549805 B1 | 02/09/2015 |
| | | KR 10-1639545 B1 | 13/07/2016 |
| | | KR 10-2010-0093575 A | 25/08/2010 |
| | | KR 10-2014-0129348 A | 06/11/2014 |
| | | KR 10-2014-0129349 A | 06/11/2014 |
| | | KR 10-2015-0100965 A | 02/09/2015 |
| | | MX 2010006390 A | 25/06/2010 |
| | | RU 2010128612 A | 20/01/2012 |
| | | RU 2465811 C2 | 10/11/2012 |
| | | TW 200926555 A | 16/06/2009 |
| | | TW I463760 B | 01/12/2014 |
| | | US 2009-0146826 A1 | 11/06/2009 |
| | | US 2012-0181991 A1 | 19/07/2012 |
| | | US 2013-0257352 A1 | 03/10/2013 |
| | | US 2016-0181842 A1 | 23/06/2016 |
| | | US 8164468 B2 | 24/04/2012 |
| | | US 8441363 B2 | 14/05/2013 |

Form PCT/ISA/210 (patent family annex) (January 2015)

INTERNATIONAL SEARCH REPORT
Information on patent family members

International application No.

**PCT/KR2016/012590**

| Patent document cited in search report | Publication date | Patent family member | Publication date |
|---|---|---|---|
| | | US 9312720 B2 | 12/04/2016 |
| | | WO 2009-075696 A1 | 18/06/2009 |
| JP 2011-101730 A | 26/05/2011 | NONE | |
| KR 10-2014-0142342 A | 11/12/2014 | AU 2013-239446 A1 | 03/10/2013 |
| | | CA 2868651 A1 | 03/10/2013 |
| | | CN 104203097 A | 10/12/2014 |
| | | EP 2833790 A1 | 11/02/2015 |
| | | JP 2015-514209 A | 18/05/2015 |
| | | US 2015-0048836 A1 | 19/02/2015 |
| | | WO 2013-144617 A1 | 03/10/2013 |

Form PCT/ISA/210 (patent family annex) (January 2015)